# EUROPEAN PATENT APPLICATION

(11) **EP 4 227 955 A1**
(43) Date of publication of application: **16.08.2023**
(21) Application number: 22156666.4
(22) Date of filing: 14.02.2022
(51) Int. Cl.: G16H 30/20, G16H 30/40, G16H 50/20, A61C 9/00

(54) **ENHANCED SCANNING**

(71) Applicant: DENTSPLY SIRONA Inc., York, PA 17401 (US); Sirona Dental Systems GmbH, 64625 Bensheim (DE)
(72) Inventor: Schneider, Sascha, 64625 Bensheim (DE)
(74) Representative: Özer, Alpdeniz

(57) **Abstract**

The present teachings relate to a method for improving data acquisition during digital impression taking, comprising: performing the data acquisition by acquiring, as one or more datasets, surface data of an oral anatomy; recording, during the data acquisition, temperature data and/or chemical composition data of the oral anatomy; augmenting, to the surface data, the temperature data and/or the chemical composition data to generate multimodal intraoral data. The present teachings also relate to a software product, a system, a use and storage media.

## Description

### TECHNICAL FIELD

The present teachings relate generally to computer-implemented methods and systems for dental scanning, more particularly intraoral scanning and diagnosis assistance.

### BACKGROUND

Dental scanners such as intraoral dental scanners, or simply called intraoral scanners, are used for taking digital dental impressions of a patient. An impression is usually captured in the form of a three-dimensional ("3D") model and may relate to features of interest of oral anatomy, such as teeth and/or soft and/or hard tissues in the patient's oral cavity. The impression may be digitally stored and/or transmitted to a dental practitioner. The digital impression can also be used for other purposes, such as producing a dental restoration and planning or performing a dental treatment.

It is known to use intraoral scanners based on structured light principle. Such scanners comprise means for projecting light patterns on the intraoral geometry. The pattern when projected on intraoral geometry appears contoured dependent upon the shape of the geometry. The scanners also comprise a camera for capturing light reflected from the geometries which comprises information related to the contours formed in the projected pattern. Signal from the camera is then sent to a computing unit for extracting shape information of the intraoral geometry. Subsequently, a digital impression or 3D model of the intraoral geometry can be reconstructed from the shape information, which usually is in the form of one or more point-clouds. Additional or alternative shape representations such as meshes can also be generated. The point cloud or mesh comprises data usually from a plurality of datasets which are captured during the scan. It shall be appreciated that the datasets are obtained in response to the signal from the camera.

In recent times, self-scanning or self-use type dental scanners have also been introduced. Such scanners can allow a patient to scan their own oral cavity, or in general allow non-practitioners to perform a scanning operation. This can save costs for the user as compared to having a dental practitioner or professional perform the scan and/or dental health monitoring can be improved by performing more regular and/or faster scans at home. For direct to customer market, lower cost solutions are generally more attractive. Such scanners can also be attractive for remotely provided health services, or so-called telehealth service.

Even for professionally generated digital impressions, it is possible that the scan has been performed by a different professional as compared to the dental practitioner receiving the digital impression. Thus, it is possible that the dental practitioner is located remotely from the professional who performed the scan on the patient. As discussed, in some cases such as telehealth, even the patient may be located remotely from the dental practitioner.

The applicant has recognized a need for improved intraoral scanning methods and devices, especially such methods and devices which may enhance digital impression taking.

### SUMMARY

At least some of the limitations associated with the foregoing can be overcome by the subject matter of the accompanying independent claims. At least some of the additional advantageous alternatives will be outlined in the dependent claims.

The applicant has realized by inventive effort that digital impressions and/or their associated scan data can be enriched with multimodal intraoral data, which can be helpful in providing better service and/or diagnosis for the patient. Even further synergistically, the data obtained pursuant to the present teachings can be used to advantageously leverage data-driven techniques to improve patient diagnosis and/or treatment.

More specifically, when viewed from a first perspective, there can be provided a computer-implemented method for improving data acquisition during digital impression taking, which method comprises:
- performing the data acquisition by acquiring, as one or more datasets, surface data of an oral anatomy;
- recording, during the data acquisition, temperature data and/or chemical composition data of the oral anatomy;
- augmenting, to the surface data, the temperature data and/or the chemical composition data to generate multimodal intraoral data.

Thus, while performing an intraoral scan, temperature and/or chemical composition data of the same oral anatomy are also recorded. These data are augmented to the surface data while capturing of which their corresponding temperature and/or chemical composition data were recorded. This results in the multimodal intraoral data, which can be seamlessly generated while performing a usual dental scan of the patient. The multimodal intraoral data represent highly enriched data which can be very valuable for use in data-driven solutions. As compared to manually tagged data, the multimodal intraoral data can be more accurate and require significantly lower effort to produce. Especially in intraoral scans, an intraoral scanner is usually already inserted and navigated in the patient's oral anatomy, this can be leveraged to also collect the temperature data and/or the chemical composition data, preferably locally at the specific location where the scanner is at a given time capturing a given scan dataset.

The multimodal intraoral data can be leveraged for different purposes, for example the method may comprise:
- analyzing at least a portion of the multimodal intraoral data;
- determining, from the analysis, at least one diagnosis probability value.

Thus, the enriched and properly contextualized nature of the multimodal intraoral data makes them enhanced in capability and suitable for determining at least one diagnosis probability value. Such one or more diagnosis probability values can be helpful for the dental practitioner in reaching a suitable diagnosis for the patient. The multimodal intraoral data can thus also be a valuable enabling factor in the field of remote health services, by allowing a more accurate and/or relevant analysis to be made of a dental condition of the patients.

Preferably, at least a part of the analysis is performed via a diagnosis data-driven logic. Since the multimodal intraoral data are relevantly augmented surface data with their corresponding temperature data and/or chemical composition data, it makes them especially suitable for leveraging data-driven techniques for computerized analysis. At least a part of the multimodal intraoral data can thus be provided as an input to the diagnosis data-driven logic. The diagnosis data-driven logic can thus provide at least one diagnosis probability value as an output. The at least one diagnosis probability value can for example be provided at a human machine interface ("HMI"), which may be in the form of a video and/or an audio device. The HMI can for example inform the dental practitioner about the possible condition of the patient estimated via the respective one or more diagnosis probability values.

Preferably, at least one recommendation is also automatically provided for improving the dental condition of the patient. Thus, the method may also comprise:
- recommending, in response to any of the diagnosis probability values, at least one further diagnosis and/or treatment.

This can also be valuable for the dental practitioner to reach a suitable treatment or diagnosis plan for the patient.

According to an aspect, the method also comprises:
- automatically triggering, in response to any of the diagnosis probability values, at least one diagnosis and/or treatment operation.

The process can thus be automated such that time and effort is saved. For example, in some cases, already during the scan one or more of the diagnosis probability values may be generated and communicated to the dental practitioner or professional performing the scan, this can also automatically set up additional procedure which can be performed on the patient while they are already in the chair for the scan. In some cases, the preparation for the subsequent diagnosis and/or treatment operation may be automatically triggered such that the practitioner or the patient does not need to wait for it. This can make the experience more comfortable and/or valuable for the patient and/or the practitioner.

As it was mentioned, the multimodal intraoral data can be valuable in different aspects. The multimodal intraoral data can for example be provided at one or more memory storages, for example, to be available for different purposes. Preferably, any one or more of the diagnosis probability values are also provided at any of the memory storages. Accordingly, the multimodal intraoral data can be enriched further, for example, when the data are leveraged as historical data for training future data-driven logics. Similarly, one or more diagnosis annotation data are also provided at any of the memory storages. The diagnosis annotation data may for example comprise information of the diagnosis made by the dental practitioner for a given digital impression. The diagnosis annotation data may thus comprise one or more annotations for one or more different digital impressions. This can make the plurality of multimodal intraoral data even more enriched and thus valuable for leveraging data-driven techniques.

Hence, any of the memory storages may also be provided with historical multimodal intraoral data and corresponding historical diagnosis probability values and/or diagnosis annotation data.

The temperature data may be generated via one or more temperature sensors. For example, one or more IR sensors may be used. The temperature sensor or the IR sensor preferably comprises an array of sensor elements for providing temperature data as a localized temperature distribution over a spatial area of the oral anatomy. Thus, preferably local hot or cold spots can be located, and the digital impression provided with the spatial temperature distribution. This can be especially valuable in pin-pointing pathological conditions. It shall be appreciated that for data-driven techniques, this can be another finer level of information for providing a more accurate diagnosis, e.g., by pattern detection in the multimodal intraoral data.

The chemical composition data may be generated via one or more chemical sensors, which may be in any suitable form. The chemical sensors may analyze a sample collected during the intraoral scan. The sample may be in fluid form, or in gas or vapor form. Thus, the sensors may analyze the sample in liquid, gas, or vapor form. For example, any one or more of the chemical sensors may be used to analyze or selectively detect certain chemical compositions at a particular site in the oral anatomy by collecting local saliva. Additionally, or alternatively, in a more preferable form, the chemical sensors may collect and analyze vapors or gases at a particular location in the oral anatomy. Another advantage of gas or vapor sensors can be that other underlying conditions, such as those manifested beyond the oral anatomy may be detected. For example, conditions in the throat or in anatomies located even deeper within the body may be detectable using the gas or vapor sensors. Thus, chemical compositions carried to the patient's oral anatomy via breathing may also be detected. Hence, a more overall well-being of the patient may be evaluated simultaneously while performing the intraoral scan.

The information space of the multimodal intraoral data can be further enhanced by:
- augmenting, to the surface data, additional modality data of the oral anatomy, wherein, the additional modality data comprises any one or more types of: color image data, X-ray data, and CBCT data.

Thus, the capability for providing a more accurate diagnosis can be further improved by providing multi-modality data or the additional modality data of the same patient.

The pattern detection or recognition in the multimodal intraoral data by the diagnosis data-driven logic may be done in different ways, for example, any one or more of, X-ray image density information, texture, shape or contour, distribution (e.g., temperature distribution and/or chemical distribution), and relative location. The diagnosis data-driven logic may correlate specific features of one data type with another data type for providing the at least one diagnosis probability value.

Advantageously, the method also comprises:
- augmenting, to the digital impression, at least a part of the temperature and/or the chemical composition data; and/or at least one diagnosis probability value to generate an enhanced digital impression.

Thus, the enhanced digital impression can be more useful for the dental practitioner who may get a more complete picture of the patient's dental condition rather than just viewing a 3D model of the oral anatomy. The augmenting of the data and/or the diagnosis probability values may be done for example by creating one or more layers overlaid on the digital impression. The layer may be in any suitable form which allows overlaying the data, for example, the layer may be in the form of a mesh. By providing at least one layer overlaid to the digital impression, especially in a graphical representation, the dental practitioner can view for example temperature distribution over the oral anatomy. Similarly, the same layer, or another one may be provided for one or more chemical composition distributions which were measured at different locations in the oral anatomy during the scan. Another advantage of providing layers can be that they can be switched on or off as desired by the practitioner. Also, the practitioner can decide which layer to view. Furthermore, if the practitioner adjusts the viewing angle of the digital impression on the HMI, the data layer(s) comprising the temperature and/or the chemical composition data and/or the diagnosis probability values is adjusted as well to represent data from the visible portion on the HMI. Hence, the enhanced digital impression comprising the digital impression augmented with said data can be significantly more valuable for the practitioner.

Hence, from the aforementioned, the method may also comprise:
- providing, at one or more memory storages, the digital impression and/or the enhanced digital impression.

According to an aspect, the memory storages may also be provided with historical multimodal intraoral data and corresponding historical diagnosis probability values and/or diagnosis annotation data.

By "historical" in the present context it is meant data, values or impressions respectively, from other patients or the same one, from scans or processing which have been performed prior to analyzing given multimodal intraoral data. Thus, data from earlier scans can be leveraged to progressively improve the process and devices further. Similarly, any one or more of the multimodal intraoral data, the enhanced digital impression, the diagnosis annotation data, and the diagnosis probability values may be included in the historical data for training data-driven logics for future use.

It shall be appreciated that the temperature data may be generated via one or more temperature sensors. At least one of the temperature sensors may be in the form of an IR sensor. The temperature sensor or the IR sensor comprises an array of sensor elements for providing temperature data as a localized temperature distribution over a spatial area of the oral anatomy.

According to an aspect, the method may also comprise:
- augmenting, to the surface data, additional modality data of the oral anatomy.

As a few non-limiting examples, the additional modality data may comprise any one or more types of: color image data, X-ray data, and CBCT data.

As it shall be appreciated by those skilled in the art, the datasets and logics as provided pursuant to the present teachings can be valuable for a variety of applications. Thus, when viewed from another perspective, there can also be provided a use of multimodal intraoral data and/or historical multimodal intraoral data as generated according to any of the method aspects herein disclosed as training data for training a machine learning ("ML") logic.

For example, there may be provided a method for training a machine learning logic, comprising:
- providing, as training data, the multimodal intraoral data and/or the historical multimodal intraoral data at the machine learning logic;
- training using the training data, the machine learning logic.

It shall be appreciated that therefrom the diagnosis data-driven logic and/or anatomy data-driven logic may be obtained. However, data-driven logics for other purposes may also be obtained, e.g., by combining other kinds of data in the training data.

Also, when viewed from another perspective, there can also be provided a dataset comprising the multimodal intraoral data and/or the historical multimodal intraoral data and/or the enhanced digital impression as generated according to any of the method aspects herein disclosed.

Furthermore, there can also be provided, a memory storage or a computer storage medium storing thereupon the multimodal intraoral data and/or the historical multimodal intraoral data and/or the probability values and/or the enhanced digital impression as generated according to any of the method aspects herein disclosed.

Similarly, there can also be provided, a memory storage or a computer storage medium storing a logic, at least partial computational functionality of which logic is caused by the logic being trained using the multimodal intraoral data and/or historical multimodal intraoral data as generated according to any of the method aspects herein disclosed.

When viewed from yet another perspective, there can also be provided a computer software product, or a non-transitory computer-readable storage medium storing the program, comprising instructions which when executed by suitable one or more computing units cause any of the computing units to perform the steps of any of the method aspects herein disclosed.

For example, there can be provided a computer software product, or a non-transitory computer-readable storage medium storing the program, comprising instructions which when executed by suitable one or more computing units cause any of the computing units to:
- perform the data acquisition by acquiring, as one or more datasets, surface data of an oral anatomy;
- record, during the data acquisition, temperature data and/or chemical composition data of the oral anatomy;
- augment, to the surface data, the temperature data and/or the chemical composition data to generate multimodal intraoral data.

When viewed from another perspective, there can be provided a system, or a system for improving data acquisition during digital impression taking, said system comprising means for performing the steps of any of the method aspects herein disclosed.

For example, there can be provided a system for improving data acquisition during digital impression taking, the system comprising one or more computing units, wherein any of the computing units is configured to:
- perform the data acquisition by acquiring, as one or more datasets, surface data of an oral anatomy;
- record, during the data acquisition, temperature data and/or chemical composition data of the oral anatomy;
- augment, to the surface data, the temperature data and/or the chemical composition data to generate multimodal intraoral data.

Any or all of the computing units may be operatively connected to the intraoral scanner via a connectivity interface. The connectivity interface may be part of a network interface or vice versa. Similarly, any or all of the computing units may be operatively connected to any or all memory storages. It can also be contemplated that the operative connection between at least one of the computing units and the intraoral scanner is via at least one of the memory storages. This may be the case for example when certain parts of the system are implemented as a cloud service. For example, one or more computing units and/or one or more memory storages may be part of one or more cloud services. Alternatively, or additionally, at least one of the computing units and/or at least one of the memory storages may be part of the intraoral scanner.

It shall be appreciated that at least some of the method steps may be implemented via the intraoral scanner.

"Data acquisition" in the present context refers to at least the process of capturing intraoral scan data. The scan data or surface data is obtained via a scanner in the form of one or more datasets. The data acquisition may also include one or more computerized processing operations.

"Digital impression" refers to a 3D digital model obtained via intraoral scanning of the patient's oral cavity. The digital model may include representation of the patient's teeth and/or hard and/or soft tissues in and around the patient's gums. The digital impression may be usable for providing for the patient any one or more of: dental treatment or procedure, surgical templates, custom devices, prosthetic restorations, and orthodontic aligners. The digital impression is built from surface data which for example are acquired via an intraoral scanner.

"Enhanced digital impression" refers to an augmented version of the digital impression to which at least a part of the temperature and/or chemical composition data have been augmented. Alternatively, or additionally, the enhanced digital impression may refer to an augmented version of the digital impression to which at least one diagnosis probability value has been augmented. Preferably, the augmentation associates the augmented data to a specific part or location of the oral anatomy represented by the digital impression.

"Surface data" refer to data which are used for building the digital impression. The surface data may be provided as one or more datasets which are generated during an intraoral scan.

"Oral anatomy" refers to the anatomy of the oral cavity of a patient. The oral anatomy usually includes a plurality of anatomical parts such as dentition, gingiva, soft tissue, etc. In some cases, man-made parts such as replacements or restorations such as any one or more of, dental filling, crown, bridge, braces, veneer, implant, etc. may also be present in the oral anatomy. Thus, the oral anatomy may include natural as well as artificial parts.

"Dental condition" refers more specifically to a state of the oral anatomy at the time at which the surface data are captured, or the time at which the intraoral scan is performed for providing the surface data.

"Anatomical feature" refers to information related to a specific oral anatomy of the patient. For example, an anatomical feature may be information related to a specific anatomical part such as a tooth or a group of teeth. Thus, anatomical features may even refer to information related to any one or more intraoral structures such as, dentition, gingiva, nerve channel, extraction site, jawbone, and condyle. Alternatively, or in addition, anatomical features may be one or more artificial structures such as one or more dental replacements, e.g., dental crown, braces, veneer, bridge. Alternatively, or in addition, an anatomical feature may in some cases even be a scan body (or scanbody) or other natural and/or artificial structure attached to the jaw of the patient. Alternatively, or additionally, the anatomical feature may even be information related to a pathology or condition. As non-limiting examples, pathology or condition may be any one or more of, fracture of tooth or bone, caries, radiolucency, impaction, or any other characterizable state of the oral anatomy or any part thereof. A few non-limiting examples of the anatomical features are, any one or more of: shape, outline or boundary of any one or a group of teeth, their arrangement, sequence in any direction, a presence or a lack of presence of an expected feature such as a given tooth type. Alternatively, or additionally, the anatomical features may also be a shape, region, or a spot detected within an area or interest, such as an outline or boundary. Alternatively, or additionally, the anatomical features may also be, or they may be detected via, an interrelation between two different anatomical features. For example, a boundary between a tooth and gingiva.

"Intraoral features" refers to information related to intraoral structures such as dentition and/or gingiva and/or other intraoral anatomical features of oral anatomy of a patient. Alternatively, or in addition, at least some of the intraoral structures of which intraoral features are recorded may be one or more artificial structures such as one or more dental replacements, e.g., dental crown, bridge, veneer, etc. Alternatively, or in addition, the intraoral structure may in some cases even be a scan body (or scanbody) or other natural and/or artificial structure. The intraoral features, more importantly surface data are recorded for building a digital impression of a patient's oral anatomy. Usually for producing the digital impression, structures which are rigidly attached to the jaw of the patient are of more interest.

"Intraoral scanner" or "intraoral dental scanner" refers to a device that is used to capture a digital impression of oral anatomy or dental condition. The terms may be used interchangeably and refer to a dental scanner that is usable for generating dental impressions for a patient. Thus, the intraoral scanner is usable for generating a digital model of the patient's mouth. The intraoral scanner comprises at least one image sensor for recording reflection of light which is reflected from the oral anatomy of the patient. The intraoral scanner may also comprise at least one light source for projecting light on the intraoral structures. An output of the image sensor or camera generated in response to the reflections may thus be used for providing the surface data. The output may be in the form of a data stream generated continuously or intermittently as the intraoral scanner records reflections or images from the oral anatomy of the patient.

The intraoral scanner thus records the digital impression of the dental condition by detecting features, especially geometrical features, of the oral anatomy. These intraoral features are then used to produce the digital impression.

During an intraoral scan, as the intraoral scanner is moved along the parts of the oral anatomy, the digital impression may be built progressively or in parts by stitching together surface data from different parts or locations. In other cases, the digital impression may be reconstructed after completing the scan, i.e., after acquiring the entire surface data from the scan.

"Augmenting" in the present context refers to the process of linking or establishing a relationship between specific data with another data. The process may include any one or more of: storing together, appending, creating a linkage such as a lookup table or reference, and their likes. Pursuant to the present teachings, the temperature data and/or the chemical composition data are augmented to the surface data. Preferably, augmentation is done between specific datasets. For example, a specific part of the surface data and augmented with a specific part of the temperature data and/or the chemical composition data. As a non-limiting example, the temperature data and/or the chemical composition data measured at a specific location of the oral anatomy are augmented to that part of the surface data which relates to the scan at that location. Similarly, in cases the sensors for capturing the temperature data and/or the chemical composition data are located at a different location on the intraoral scanner as compared to the camera view of the scanner, the augmentation may be done between those parts of the temperature data and/or the chemical composition data and the surface data which correspond to the same or essentially the same location. The pre-knowledge of the relative location of the camera with respect to the temperature and/or chemical sensor(s) can be used to calculate when during the scan the camera is present at a position where the temperature and/or chemical sensor(s) were or vice-versa. The relevant datasets from the camera and the temperature and/or chemical sensor(s) can thus be augmented after the data from all sensors for the same location are available during the scan.

"Multimodal intraoral data" refers to data which comprise at least a part of the surface data and at least a part of the temperature and/or chemical data which have been recorded during the same scan operation on the patient. The multimodal intraoral data are enriched by augmenting the temperature and/or chemical data with the surface data and/or the digital impression.

The multimodal intraoral data can also comprise information related to at least one anatomical feature. The anatomical features may be recorded from diagnosis annotation data and/or by automatically analyzing the digital impression via a computing unit. For example, one or more segmentation operations and/or localization operations may be performed on the digital impression. Preferably, the detection of anatomical features is also done using data-driven techniques, for example via an anatomy data-driven logic.

In some cases, the multimodal intraoral data may also comprise the digital impression of the same patient. Preferably, at least a part of the temperature data and/or the chemical composition data is annotated or augmented to the digital impression. That way, a dental practitioner can also be provided with a more comprehensive impression of the patient's anatomy. This can simplify diagnosis and thus result in a quicker resolution of pathological problems.

"Analysis" refers to computer implemented operation of analyzing at least a part of the multimodal intraoral data. Thus, any one or more computing units may be used for performing the analysis of the multimodal intraoral data or a part thereof for computing at least one diagnosis probability value. The computing unit may calculate the diagnosis probability value by detecting specific features in the multimodal intraoral data. For example, it may be evaluated presence of a particular feature in the surface data and/or the digital impression vis-à-vis a specific feature or pattern in the temperature data and/or the chemical composition data or vice-versa to calculate a specific diagnosis probability value. Thus, at least the features or patterns which may inconclusive when analyzing the surface data alone may provide useful diagnosis information when evaluated in context of the temperature and/or chemical composition data, or vice-versa. As a further non-limiting example, a detection of a particular chemical composition, e.g., a given gas and/or elevated temperature along with a particular size or shape of the gingiva may be used to provide a high diagnosis annotation data for gingivitis. Preferably, the diagnosis probability value is provided associated with a particular location or part of the oral anatomy. Further preferably, the diagnosis probability value is automatically annotated to the multimodal intraoral data, and more preferably to a specific part of the multimodal intraoral data which the diagnosis probability value relates to.

"Diagnosis probability value" refers to a value or estimate associated with a given condition of the oral anatomy. As a non-limiting example, the diagnosis probability value may be *"80% certainty of gingivitis".* In some cases, the diagnosis probability value may even be an estimate of an absence of a given condition. Thus, the diagnosis probability value may be indicative of a given dental condition or a lack thereof.

"Data-driven logic" refers to a logic, which at least partially derives its functionality from training data. In contrast to a rigorous or analytical logic which is based on programmed instructions for performing a particular logical task, a data-driven logic can allow forming such instructions at least partially automatically using the training data. The use of data-driven logic can allow to describe logical and/or mathematical relationships without solving equations. This can reduce computational power and/or improve speed. Moreover, data-driven logic may be able to detect patterns or indications (e.g., in input or input data provided to the data-driven logic) which can otherwise not be known or may be difficult to implement as an analytical logic form.

The data-driven logic may be in software and/or hardware form, for example, executable via one or more computing units.

It shall be appreciated that in the present context, the data-driven logic refers to a trained mathematical logic which is parametrized according to the respective training data set. For example, the diagnosis data-driven logic is parameterized via its respective training data to compute at least one diagnosis probability value. An untrained logic lacks this capability. Hence, the untrained logic or model is incapable for performing a desired detection. Feature engineering and training with the respective training datasets thus enables parametrization of the untrained logic. The result of such a training phase is the respective data-driven model, which as a result of the training process, preferably solely as a result of the training process, provides interrelations and logical capability related to the purpose for which the respective data-driven logic is to be used.

The data-driven logic may comprise, or it may be in the form of a regression logic. In some cases, the data-driven logic may be combined with, or it may include an analytical logic. The analytical logic may describe the relation between its input and output as a function or one or more mathematical equations or logical criteria. Thus, any of the data-driven logics described herein may even be a hybrid logic. A hybrid logic refers to a logic which comprises first-principles parts, so called white-box described via a set of equations e.g., mathematical equations, as well as data-driven parts as explained previously. The data-driven part may also be called black-box logic or model. Hence, the data-driven logic in some cases may be a combination of white-box logic or analytical logic, and black-box logic. In some cases, the data-driven logic may be, or it may even have a grey-box part. A grey-box logic in this context refers to a logic or model which combines a partial analytical structure with a data-driven part using training data to complete the model.

The diagnosis data-driven logic is trained using diagnosis training data which may comprise historical surface data and their associated temperature and/or chemical composition data. Preferably, the diagnosis training data comprise at least partly historical multimodal intraoral data. The historical diagnosis data preferably comprise respective diagnosis annotation data. The diagnosis training data may even comprise respective at least one diagnosis probability value.

Thus, the method may also comprise one or more training step for obtaining the diagnosis data-driven logic by training a mathematical logic using the diagnosis training data.

"Segmentation operation" in the present context refers to a computerized processing operation which concludes with demarcation of at least one anatomical feature with a computer-generated feature boundary.

The anatomy data-driven logic may perform a segmentation operation for detecting the one or more pre-selected anatomical features. Thus, the anatomy data-driven logic may segment at least one of the projection images for detection. Thus, the anatomy data-driven logic may extract attributes or one or more objects of interest from at least one of the projection images. The anatomy data-driven logic may classify every pixel in the respective projection image to a class according to its context such that each pixel is assigned to a specific object. A non-limiting example of a segmentation operation when applied using the present teachings may be an outline around a pre-selected anatomical feature, such as a specific tooth or even a group of teeth. Another non-limiting example is an outline around a specific pathology. Said segmented outline corresponds to the shape of the respective anatomical feature as visible in the respective projection image.

There may be a plurality of segmented objects in any given projection image.

In addition to the data-driven approach, the segmentation operation may be supplemented by an analytical model using any suitable segmentation algorithm, e.g., point and line detection, edge linking, and/or thresholding method, histogram, adaptive, and their likes.

A "localization" operation in the present context refers to a computerized processing operation which concludes with a more general region or location within which a specific anatomical feature is located in a projection image. The localization may also result in a boundary within with said anatomical feature is located, however, unlike a segmentation operation, a pixel-by-pixel evaluation is not performed. Hence, a localization boundary, if generated, may be broader than a segmented boundary around a given anatomical feature. Additionally, or alternatively, a localization operation may conclude with annotating or tagging a specific anatomical feature, for example in any location on said anatomical feature.

Similarly, in addition to the data-driven approach, the localization operation may be supplemented by an analytical model using any suitable localization algorithm, e.g., edge and shape detection, and their likes.

"Human machine interface" or "HMI" refers to a device or system which comprises at least one video unit or display screen and/or audio device for providing to the user the diagnosis probability value.

Thus, the HMI system may comprise a visual display or screen for displaying visual images or video. Alternatively, or additionally, the HMI system may comprise an audio device such as a loudspeaker for outputting audio. The video unit may comprise a video screen which may be active or passive, for example an LCD screen, OLED screen or projection based video system. Alternatively, or additionally, the video unit may comprise an augmented reality ("AR") device.

The HMI may even comprise one or more input devices for receiving user input.

"Further diagnosis and/or treatment" refers to any follow-up diagnosis or treatment operation which may be deemed beneficial for confirming a particular diagnosis and/or treating the diagnosed condition of the patient.

"Additional modality data" related to given surface data or multimodal intraoral data refer to data from other medical scans than an intraoral scan, although from the same patient. For example, the additional modality data may comprise any one or more of: X-ray data, CBCT data, image data such as color images, etc.

"Anatomy data-driven logic" may refer to a data-driven logic which is trained or pretrained in a supervised manner using anatomy training data comprising a plurality of historical digital impressions with annotation data specifying respective anatomical features. Alternatively, or additionally, the anatomy data-driven logic may be trained in an unsupervised manner.

The anatomy data-driven logic may be provided with the digital impression as input. The anatomy data-driven logic may provide anatomy data as an output. The anatomy data may thus comprise information related to one or more anatomical features.

According to an aspect, the anatomy data-driven logic performs segmentation operations for detecting the one or more pre-selected anatomical features. Alternatively, or additionally, the anatomy data-driven logic performs detection and/or localization operations for detecting the one or more pre-selected anatomical features.

Thus, in general, the anatomy data-driven logic extracts features of interest, or the pre-selected anatomical features, from the digital impression.

In some cases, the anatomy data-driven logic and the diagnosis data-driven logic may be the same logic, for example a combined logic which is a data-driven logic for performing the detection of one or more anatomical features as well as providing at least one diagnosis probability value. Thus, in such a case, the multimodal intraoral data preferably also comprising the digital impression may be provided via an input of the combined logic, and the at least one diagnosis probability value may be provided via an output of the combined logic, preferably the diagnosis probability value is annotated to a specific anatomical feature or a group of anatomical features in the digital impression.

It shall be appreciated that an automatic annotation to a specific anatomical feature or a group of anatomical features in the digital impression can also be provided when the anatomy data-driven logic and the diagnosis data-driven logic are implemented separately. An advantage of providing said automatic annotations on the digital impression can be that the dental practitioner can directly be pointed to where a certain pathology is expected. This can be of significant assistance to the practitioner in reaching a suitable diagnosis for the patient. This can also help allow catching certain pathological problems early on, for example an additional issue which may not be the main reason why the practitioner was provided the digital impression for.

"Combined logic" refers to a logic comprising at least of the two different data-driven logics disclosed herein. The combined logic may be pretrained in a supervised manner using an end-to-end training process. The end-to-end training process in this context refers to at least partially annotated data comprising inputs and corresponding outputs. In some cases, the combined logic may be trained fully or partially in an unsupervised manner.

"Memory storage" may refer to a device for storage of information, in the form of data, in a suitable storage medium. Preferably, the memory storage is a digital storage suitable for storing the information in a digital form which is machine readable, for example digital data that are readable via a computer processor. The memory storage may thus be realized as a digital memory storage device that is readable by a computer processor. Further preferably, the memory storage on the digital memory storage device may also be manipulated by a computer processor. For example, any part of the data recorded on the digital memory storage device may be written and or erased and or overwritten, partially or wholly, with the new data by the computer processor.

"Computing unit", "computing device", "processing unit" or "processing device" may comprise, or it may be, a processing means or computer processor such as a microprocessor, microcontroller, or the likes, having one or more computer processing cores.

"Computer processor" refers to an arbitrary logic circuitry configured for performing basic operations of a computer or system, and/or, generally, to a device which is configured for performing calculations or logic operations. In particular, the processing means or computer processor may be configured for processing basic instructions that drive the computer or system. As an example, the processing means or computer processor may comprise at least one arithmetic logic unit ("ALU"), at least one floating point unit ("FPU"), such as a math coprocessor or a numeric coprocessor, a plurality of registers, specifically registers configured for supplying operands to the ALU and storing results of operations, and a memory, such as an L1 and L2 cache memory. In particular, the processing means or computer processor may be a multi core processor. Specifically, the processing means or computer processor may be or may comprise a central processing unit ("CPU"). The processing means or computer processor may be a complex instruction set computing ("CISC") microprocessor, reduced instruction set computing microprocessor ("RISC"), Very long instruction word ("VLIW") microprocessor, a processor implementing other instruction sets or processors implementing a combination of instruction sets. The processing means may also be one or more special purpose processing devices such as an application specific integrated circuit ("ASIC"), a field programmable gate array ("FPGA"), a complex programmable logic device ("CPLD"), a digital signal processor ("DSP"), a network processor, or the like. The methods, systems and devices disclosed herein may be implemented as software in a DSP, in a microcontroller, or in any other side processor such as hardware unit within an ASIC, CPLD, or FPGA. It is to be understood that the term processing means or processor may also refer to one or more processing devices, such as a distributed system of processing devices located across multiple computer systems (such as cloud computing), and is not limited to a single device unless otherwise specified.

"Connectivity interface" or "communication interface" refers to a software and/or hardware interface for establishing communication such as transfer or exchange of signals or data. The communication may either be wired, or it may be wireless. Connectivity interface is preferably based on or it supports one or more communication protocols. The communication protocol may be a wireless protocol, for example: short distance communication protocol such as Bluetooth^{®}, or Wi-Fi, or long communication protocols such as cellular or mobile network, for example, second generation cellular network ("2G"), 3G, 4G, long term evolution ("LTE"), or 5G. Alternatively, or in addition, the connectivity interface may even be based on proprietary short distance or long distance protocol. The connectivity interface may support any one or more standards and/or proprietary protocols.

"Network" discussed herein may be any suitable kind of data transmission medium, wired, wireless, or their combination. A specific kind of network is not limiting to the scope or generality of the present teachings. The network can hence refer to any suitable arbitrary interconnection between at least one communication end point to another communication end point. Network may comprise one or more distribution points, routers or other types of communication hardware. The interconnection of the network may be formed by means of physically hard wiring, optical and/or wireless radio frequency ("RF") methods. The network specifically may be, or it may comprise, a physical network fully or partially made by hard wiring, such as a fiber optical network or a network fully or partially made by electrically conductive cables or a combination thereof. The network may at least partially comprise the Internet.

"Network interface" refers to a device or a group of one or more hardware and/or software components that allow an operative connection with the network.

That two or more components are "operatively" coupled or connected shall be clear to those skilled in the art. In a non-limiting manner, this means that there may be at least one communicative connection between the coupled or connected components e.g., they are the network interface or any suitable interface. The communicative connection may either be fixed, or it may be removable. Moreover, the communicative connection may either be unidirectional, or it may be bidirectional. Furthermore, the communicative connection may be wired and/or wireless. In some cases, the communicative connection may also be used for providing control signals.

### BRIEF DESCRIPTION OF THE SEVERAL VIEWS OF THE DRAWINGS

Certain aspects of the present teachings will now be discussed with reference to the accompanying drawings that explain said aspects by the way of examples. Since the generality of the present teachings is not dependent on it, the drawings may not be to scale. Method and system aspects may be discussed in conjunction for ease of understanding. Certain features shown in the drawings may be logical features that are shown together with physical features for the sake of understanding and without affecting the generality or scope of the present teachings.
FIG. 1 illustrates a block diagram of a scanning system;
FIG. 2 illustrates a flowchart 200 showing a method aspect of the present teachings.

### DETAILED DESCRIPTION

In accordance with example aspects described herein, methods, systems and computer readable storage media can be provided, e.g., for improving data acquisition during digital impression taking.

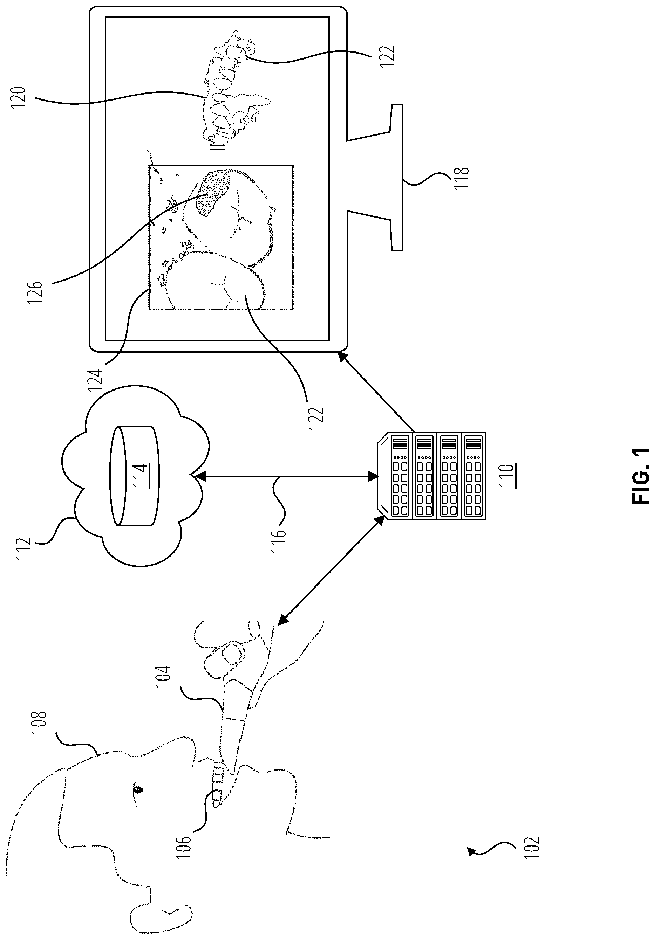

FIG. 1 shows a block diagram 102 of system. The system in this specific case is shown comprising an intraoral scanner 104, which is shown in FIG. 1 being used for digital impression taking of a patient 108. The intraoral scanner 104 is operatively connected to a computing module 110, which comprises at least one computing unit. In some cases, the computing module 110 may be located at least partially within the intraoral scanner 104. In some cases, the computing module 110 may at least partially be a part of a cloud service 112. Thus, the computing module 110 may be a local device at the location of the intraoral scanner 104 and/or at least partially a remotely located device or system, e.g., one or more cloud services 112 and/or a remote server. The computing module 110 in this example is also operatively connected to a memory storage 114, which may at least partially be a part of the cloud service 112. In this case, the memory storage 114 is shown as a part of the cloud service 112. The intraoral scanner 104 may connect to an external computing module 110 via a connectivity interface (not explicitly shown in FIG. 1). Any local devices, e.g., the intraoral scanner 104 and/or the local computing module 110 may connect to the cloud service 112 via one or more networks 116. A network interface (not explicitly shown in FIG. 1) may be used to connect the local devices to the remote devices, e.g., the cloud service 112. In some cases, the network interface and the connectivity interface may be the same unit.

The computing module 110 is operatively connected to a human machine interface 118 ("HMI"), which in FIG. 1 is shown as a computer screen. It is possible that the HMI 118 is located remotely from the location where the scanning is being performed. It is also possible that the HMI 118 is displayed/viewed at another time, i.e., not concurrently during the operation, e.g., after completing the intraoral scan of the patient 108. For example, the HMI 118 may be related to a different dental practitioner than the one who performed the intraoral scan.

The connection between the computing module 110 and the HMI 118 may be via an output interface (not explicitly shown in FIG. 1), which may for example be a computer port or bus, e.g., display port, HDMI, USB, or their like. However, the output interface may even be an API for providing data to one or more computing units on the HMI 118 side. In some cases, the output interface may be a part of the network interface and/or the connectivity interface. Accordingly, it is possible that the three interfaces are the same device.

The intraoral scanner 104 comprises a sensor unit or camera (not explicitly shown in FIG. 1) for receiving reflection of the surface of the oral anatomy 106. Surface data are generated in the form of one or more datasets in response to receiving the reflection. During the scan, the intraoral scanner 104 is operated to capture the plurality images of the oral anatomy 106. The images may be in the form of a data stream which constitute the surface data. In addition to the images or surface data, the intraoral scanner 104, or an acquisition unit related to the scanner, also captures or records temperature and/or chemical composition data at the oral anatomy 106. The acquisition unit may comprise one or more sensors for measuring temperature and/or chemical composition.

The surface data and the temperature and/or chemical composition data are provided to the computing module 110. The computing module 110 is configured to augment to the surface data, the temperature data and/or the chemical composition data to generate multimodal intraoral data. The surface data may be used for building or reconstructing a digital impression 120 of the patient's oral anatomy 106.

Optionally, the computing module 110 may analyze at least a portion of the multimodal intraoral data and generate in response at least one diagnosis probability value. Advantageously, the analysis is performed using a diagnosis data-driven logic.

Any or all of the data may be provided at one of more of the memory storages 114. For example, the multimodal intraoral data may be stored thereupon for training one or more data-driven logics or machine learning models. An enhanced digital impression may also be provided at the memory storage 114, which comprises a digital impression 120 augmented with at least a part of the temperature and/or chemical composition data and/or one or more of the diagnosis probability values.

On the HMI 118 side, the same dental practitioner or another one may access the digital impression 120 of the patient 108 in a graphical form. The digital impression or a part thereof may also be available in other forms, for example, any of those forms suitable for manufacturing a dental restoration, replacement or part for the patient 108. An advantage of the present teachings is that an enhanced digital impression 124 can also be provided for the dental practitioner with which the practitioner may obtain information not only of anatomical parts 122 of the oral anatomy 106, but also temperature and/or chemical composition data 126 recorded for the specific parts of the oral anatomy 106. Similarly, the diagnosis probability values may also be shown using the enhanced digital impression 124. This can be especially valuable for remote health applications. Although the enhanced digital impression 124 is shown as a zoomed-in view of the digital impression 120 in this example, it may also be viewable as a more complete view as for the digital impression 120. It shall be appreciated that the teachings may also be deployed as a real-time scenario. An advantage in that case can be that the health practitioner can visualize the enhanced digital impression 124 during the scan itself. Moreover, the practitioner may also be informed during the scan about the diagnosis probability within the same session. This can help reaching a correct diagnosis or treatment quicker.

In any case, optionally, the multimodal intraoral data and/or the enhanced digital impression 124 may also be augmented with other statistical data. The statistical data may for example be any one or more of: average, limit values or any other statistical value or range related to the respective diagnosis probability value. As a non-limiting example, the practitioner may be provided with information, such as "an average value of this sensor is for most of the patients in the age / in this health condition is *xyz".* Where xyz may be a number or range for a specific condition.

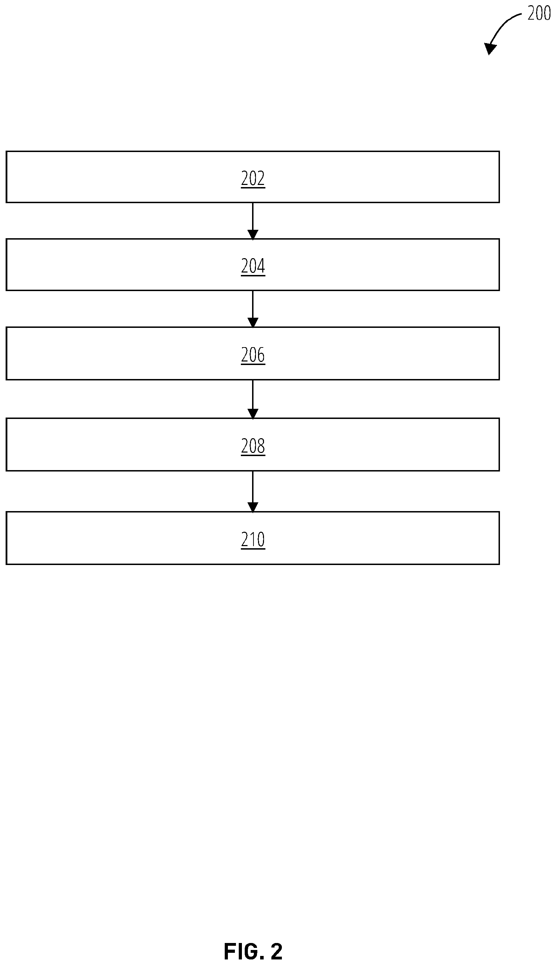

FIG. 2 shows a flowchart 200 which may be implemented as one or more routines which are executed by one or more computing units. At least certain related system aspects shall also be apparent from the following. The flowchart 200 may be implemented as one or more routines in a system, such as an intraoral scanning system.

In block 202, it is performed data acquisition by acquiring, as one or more datasets, surface data of an oral anatomy 106.

In block 204, it is recorded during the data acquisition, temperature data and/or chemical composition data of the oral anatomy.

In block 206, it is augmented to the surface data, the temperature data and/or the chemical composition data to generate multimodal intraoral data.

Optionally, in block 208, at least a portion of the multimodal intraoral data are analyzed.

Further optionally, in block 210, it is determined, from the analysis, at least one diagnosis probability value.

The method steps may be performed in the order as shown listed in the examples or aspects. It should be noted, however, that under specific circumstances a different order may also be possible. Further, it is also possible to perform one or more of the method steps once or repeatedly. These steps may be repeated at regular or irregular time periods. Further, it is possible to perform two or more of the method steps simultaneously or in a timely overlapping fashion, specifically when some or more of the method steps are performed repeatedly. The method may comprise further steps which are not listed.

The word "comprising" does not exclude other elements or steps, and the indefinite article "a" or "an" does not exclude a plurality. A single processing means, processor or controller or other similar unit may fulfill the functions of several items recited in the claims. The mere fact that certain measures are recited in mutually different dependent claims does not indicate that a combination of these measures cannot be used to advantage. Any different signs in the claim should not be construed as limiting the scope.

Further, it should be noted that in the present disclosure, the terms "at least one", "one or more" or similar expressions indicating that a feature or element may be present once or more than once typically may have been used only once when introducing the respective feature or element. Thus, in some cases unless specifically stated otherwise, when referring to the respective feature or element, the expressions "at least one" or "one or more" may not have been repeated, notwithstanding the fact that the respective feature or element may be present once or more than once.

Further, the terms "preferably", "more preferably", "particularly", "more particularly", "specifically", "more specifically" or similar terms are used in conjunction with optional features, without restricting alternative possibilities. Thus, any features introduced by these terms are optional features and are not intended to restrict the scope of the claims in any way. The present teachings may, as those skilled in the art will recognize, be performed by using alternative features. Similarly, the features introduced by "according to an aspect" or similar expressions are intended to be optional features, without any restriction regarding alternatives to the present teachings, without any restrictions regarding the scope of the present teachings and without any restriction regarding the possibility of combining the features introduced in such way with other optional or non-optional features of the present teachings.

Unless otherwise defined, all technical and scientific terms used herein have the same meaning as commonly understood by one of ordinary skill in the art to which the present teachings belong.

Various examples have been disclosed above for a method, a system, a device, a use, software program, and a computing unit comprising the computer program code for carrying out the methods herein disclosed. For example, it has been disclosed a method for improving data acquisition during digital impression taking, comprising: performing the data acquisition by acquiring, as one or more datasets, surface data of an oral anatomy; recording, during the data acquisition, temperature data and/or chemical composition data of the oral anatomy; augmenting, to the surface data, the temperature data and/or the chemical composition data to generate multimodal intraoral data. It is also disclosed at least one software product, system, use, and storage media. Those skilled in the art will understand however that changes and modifications may be made to those examples without departing from the spirit and scope of the accompanying claims and their equivalence. It will further be appreciated that aspects from the method and product embodiments discussed herein may be freely combined.

Any headings utilized within the description are for convenience only and have no legal or limiting effect.

Summarizing and without excluding further possible embodiments, certain example embodiments of the present teachings may be summarized in the following clauses:
Clause 1. A computer-implemented method for improving data acquisition, particularly intraoral data acquisition, during digital impression taking, which method comprises:
   - performing the data acquisition by acquiring, as one or more datasets, surface data of an oral anatomy;
   - recording, during the data acquisition, temperature data and/or chemical composition data of the oral anatomy;
   - augmenting, to the surface data, the temperature data and/or the chemical composition data to generate multimodal intraoral data.
Clause 2. The method of clause 1, further comprising:
   - analyzing at least a portion of the multimodal intraoral data;
   - determining, from the analysis, at least one diagnosis probability value.
Clause 3. The method of clause 2, wherein at least a part of the analysis is performed via a diagnosis data-driven logic.
Clause 4. The method of any one of clauses 2 to 3, further comprising:
   - providing, at a human machine interface, the at least one diagnosis probability value.
Clause 5. The method of any one of clauses 2 to 4, further comprising:
   - recommending, in response to any of the diagnosis probability values, at least one further diagnosis and/or treatment.
Clause 6. The method of any one of clauses 2 to 5, further comprising:
   - automatically triggering, in response to any of the diagnosis probability values, at least one diagnosis and/or treatment operation.
Clause 7. The method of any one of clauses 1 to 6, further comprising:
   - augmenting, to the digital impression, at least a part of the temperature and/or the chemical composition data; and/or at least one diagnosis probability value to generate an enhanced digital impression.
Clause 8. The method of any one of clauses 1 to 7, further comprising:
   - providing, at one or more memory storages, the multimodal intraoral data.
Clause 9. The method of any one of clauses 1 to 8, further comprising:
   - providing, at one or more memory storages, the digital impression and/or the enhanced digital impression.
Clause 10. The method of clause 8 or 9, further comprising:
   - providing, at any of the memory storages, any one or more of the diagnosis probability values.
Clause 11. The method of any one of clauses 8 to 10, further comprising:
   - providing, at any of the memory storages, diagnosis annotation data.
Clause 12. The method of any one of clauses 8 to 11, wherein any of the memory storages are also provided with historical multimodal intraoral data and corresponding historical diagnosis probability values and/or diagnosis annotation data.
Clause 13. The method of any one of clauses 1 to 12, wherein the temperature data are generated via at least one temperature sensor, preferably one of the temperature sensors being an IR sensor.
Clause 14. The method of clause 13, wherein the temperature sensor or the IR sensor comprises an array of sensor elements for providing temperature data as a localized temperature distribution over a spatial area of the oral anatomy.
Clause 15. The method of any one of clauses 1 to 14, further comprising:
   - augmenting, to the surface data, additional modality data of the oral anatomy, wherein, the additional modality data comprises any one or more types of: color image data, X-ray data, and CBCT data.
Clause 16. Use of multimodal intraoral data and/or historical multimodal intraoral data as generated in any of the above method clauses as training data for training a machine learning logic.
Clause 17. A dataset comprising the multimodal intraoral data and/or the historical multimodal intraoral data and/or the enhanced digital impression as generated in any of the above method clauses.
Clause 18. A memory storage or a computer storage medium storing the multimodal intraoral data and/or the historical multimodal intraoral data and/or the probability values and/or the enhanced digital impression as generated in any of the above method clauses.
Clause 19. A logic or a memory storage, or a computer storage medium storing the logic, at least partial computational functionality of which logic is caused by the logic being trained using the multimodal intraoral data and/or historical multimodal intraoral data as generated in any of the above method clauses.
Clause 20. A computer software program, or a non-transitory computer-readable storage medium storing the program, comprising instructions which when executed by suitable one or more computing units cause any of the computing units to perform the steps of any of the above method clauses.
Clause 21. A system comprising means for performing the steps of any of the above method clauses.
Clause 22. A system for improving data acquisition, particularly intraoral data acquisition, during digital impression taking, the system comprising one or more computing units, wherein any of the computing units is configured to:
   - perform the data acquisition by acquiring, as one or more datasets, surface data of an oral anatomy;
   - record, during the data acquisition, temperature data and/or chemical composition data of the oral anatomy;
   - augment, to the surface data, the temperature data and/or the chemical composition data to generate multimodal intraoral data.

## Claims

1. A computer-implemented method for improving data acquisition during digital impression taking, which method comprises:
- performing the data acquisition by acquiring, as one or more datasets, surface data of an oral anatomy;
- recording, during the data acquisition, temperature data and/or chemical composition data of the oral anatomy;
- augmenting, to the surface data, the temperature data and/or the chemical composition data to generate multimodal intraoral data.

2. The method of claim 1, further comprising:
- analyzing at least a portion of the multimodal intraoral data;
- determining, from the analysis, at least one diagnosis probability value.

3. The method of claim 2, further comprising:
- recommending, in response to any of the diagnosis probability values, at least one further diagnosis and/or treatment.

4. The method of any one of claims 2 or 3, further comprising:
- automatically triggering, in response to any of the diagnosis probability values, at least one diagnosis and/or treatment operation.

5. The method of any one of claims 1 to 4, further comprising:
- augmenting, to the digital impression, at least a part of the temperature and/or the chemical composition data; and/or at least one diagnosis probability value to generate an enhanced digital impression.

6. The method of any one of claims 1 to 5, further comprising:
- providing, at one or more memory storages, the multimodal intraoral data.

7. The method of any one of claims 1 to 6, further comprising:
- providing, at one or more memory storages, the digital impression and/or the enhanced digital impression.

8. The method of claim 6 or 7, further comprising:
- providing, at any of the memory storages, any one or more of the diagnosis probability values.

9. The method of any one of claims 6 to 8, further comprising:
- providing, at any of the memory storages, diagnosis annotation data.

10. The method of any one of claims 6 to 9, wherein any of the memory storages are also provided with historical multimodal intraoral data and corresponding historical diagnosis probability values and/or diagnosis annotation data.

11. The method of any one of claims 1 to 10, wherein the temperature data are generated via at least one temperature sensor, preferably one of the temperature sensors being an IR sensor.

12. The method of any one of claims 1 to 11, further comprising:
- augmenting, to the surface data, additional modality data of the oral anatomy, wherein, the additional modality data comprises any one or more types of: color image data, X-ray data, and CBCT data.

13. Use of multimodal intraoral data and/or historical multimodal intraoral data as generated in any of the above method claims as training data for training a machine learning logic.

14. A dataset, or a memory storage or a computer storage medium storing the dataset, comprising: the multimodal intraoral data and/or the historical multimodal intraoral data and/or the probability values and/or the enhanced digital impression as generated in any of the above method claims.

15. A logic or a memory storage, or a computer storage medium storing the logic, at least partial computational functionality of which logic is caused by the logic being trained using the multimodal intraoral data and/or historical multimodal intraoral data as generated in any of the above method claims.

16. A computer software program, or a non-transitory computer-readable storage medium storing the program, comprising instructions which when executed by suitable one or more computing units cause any of the computing units to perform the steps of any of the above method claims.

17. A system comprising means for performing the steps of any of the above method claims.
